# EUROPEAN PATENT SPECIFICATION

(11) **EP 4 436 643 B1**
(45) Date of publication and mention of the grant of the patent: **08.04.2026**
(21) Application number: 22817367.0
(22) Date of filing: 21.11.2022
(51) Int. Cl.: A61M 16/00, A61M 16/06, A61M 16/12, A61M 16/16, A61B 5/08

(54) **END TIDAL CARBON DIOXIDE MEASUREMENT DURING HIGH FLOW OXYGEN THERAPY**
ENDTIDALKOHLENDIOXIDMESSUNG WÄHREND EINER SAUERSTOFFTHERAPIE MIT HOHEM DURCHFLUSS
MESURE DE DIOXYDE DE CARBONE DE FIN D'EXPIRATION PENDANT UNE OXYGÉNOTHÉRAPIE À HAUT DÉBIT

(30) Priority: 23.11.2021 US 202163282335 P; 16.11.2022 US 202218056147
(43) Date of publication of application: 02.10.2024
(73) Proprietor: Covidien LP, Mansfield, MA 02048 (US)
(72) Inventor: MILNE, Gary, Minneapolis, Minnesota 55432 (US); POZDERAC, Nicholas D., Minneapolis, Minnesota 55432 (US)
(74) Representative: Marks & Clerk LLP
(86) International application number: PCT/IB2022/061221
(87) International publication number: WO 2023/094966

(56) References cited:
- WO-A1-2016/035035
- WO-A1-2018/134821
- US-A1- 2011 009 763
- US-A1- 2021 016 040

## Description

### INTRODUCTION

Medical ventilator systems have long been used to provide ventilatory and supplemental oxygen support to patients. These ventilators typically comprise a connection for pressurized gas (air, oxygen) that is delivered to the patient through a conduit or tubing. One example of such ventilation is known as high-flow oxygen therapy (HFOT). Where nasal cannulas are utilized for the patient interface, such therapy may also be known as high-flow nasal cannula (HFNC) oxygen therapy. During HFOT, a patient is provided a continuous, relatively high flow of breathing gases that include an increased concentration of oxygen. For example, some HFOT systems are capable of delivering flow rates of up to 100 liters per minute (lpm) with oxygen concentrations (e.g., fraction of inspired oxygen (FiO₂) levels) of up to or above 90% or 100%.

WO2016/035035A1 discloses a method of determining a corrected exhaled gas measurement during high flow respiratory therapy.

US2011009763A1 discloses a system for sampling exhaled breath and for supply of a gas, the system comprising: a gas delivery cannula comprising at least one nasal prong for insertion into a nostril, the nasal prong comprising a distal end; an exhaled breath sampling cannula for insertion into the nostril, the exhaled breath sampling cannula comprising a distal end; and a connector for coupling the gas delivery cannula to the exhaled breath sampling cannula, such that the distal end of the exhaled breath sampling cannula is disposed deeper in the nostril than the distal end of the nasal prong, to reduce dilution of sampled exhaled breath by delivered gas.

US2021016040A1 discloses a method of method of high flow oxygen therapy (HFOT) and carbon dioxide (CO₂) monitoring including delivering high flow oxygen therapy (HFOT) via a central lumen of a nasal cannula, the nasal cannula comprising a proximal end, a distal end positioned within a pharynx region of a patient's airway, and the central lumen and a sampling lumen formed within a wall of the nasal cannula.

It is with respect to this general technical environment that aspects of the present technology disclosed herein have been contemplated. Furthermore, although a general environment is discussed, it should be understood that the examples described herein should not be limited to the general environment identified herein.

### SUMMARY

The invention is defined by the appended claims. This summary is provided to introduce a selection of concepts in a general and/or simplified form that are further described below in the Detailed Description.

Among other things, aspects of the present disclosure include systems and methods for capturing capnography data during a temporary reduction in flow during high flow oxygen therapy. In an aspect, the technology relates to a method for capturing capnography data, for example during high-flow oxygen therapy (HFOT). The method includes delivering breathing gases at an operational flow rate and an operational oxygen concentration level, for example thereby delivering HFOT; initiating a temporary flow-reduction function for a set duration, wherein breathing gases are delivered at a temporary flow rate for the set duration, the temporary flow rate being less than the operational flow rate; capturing capnography data for exhaled air during the set duration; and upon expiration of the set duration, resuming delivery, for example HFOT delivery.

In an example, the temporary flow rate is at least 20 liters per minute (lpm) less than the operational flow rate. In another example, initiating the temporary flow-reduction function is performed by the ventilator. In a further example, initiating the temporary flow-reduction function is performed by a capnography monitor. In still another example, the capnography data is captured by the capnography monitor. The method may further comprise boosting an oxygen concentration level for at least 1 breath immediately prior to reducing the flow rate. In yet another example, during the set duration, the breathing gases are delivered at a temporary oxygen concentration level that is greater than the operational oxygen concentration level. In still yet another example, based on the captured capnography data, automatically adjusting the temporary flow rate or the set duration for a subsequent temporary flow-reduction function.

In another example, the set duration is at least 3 breaths. In still another example, the operational flow rate is at least 40 lpm. In yet another example, the method further includes calculating an end-tidal carbon dioxide value from the captured capnography data. The method may comprise generating capnography trend data from the capnography data and, based on the capnography trend data being outside a capnography threshold, activating a notification.

In another aspect, which may be provided independently, the technology relates to a system for capturing capnography data, for example during high-flow oxygen therapy (HFOT). The system includes a valve coupled to ventilation tubing, the valve configured to alter a flow of breathing gases delivered to a patient from a ventilator; and a capnography monitor communicatively coupled to the valve. The capnography monitor includes a processor; and memory storing instructions that, when executed by the processor, cause the capnography monitor to perform a set of operations. The operations include at least partially close the valve for a set duration to reduce a flow rate of breathing gases delivered to the patient; during the set duration, capturing capnography data for exhaled air; and upon expiration of the set duration, reopening the valve to allow the flow rate of breathing gases delivered to the patient to increase.

In an example, capturing capnography data comprises sampling the exhaled air at a sampling interface and drawing the air into the capnography monitor through a sampling line. In another example, the operations further comprise comparing the capnography data to a capnography threshold, and activating a notification when the capnography data exceeds the capnography threshold. In a further example, at least partially closing the valve reduces the flow rate of breathing gases by at least 20 liters per minute. In still another example, the set duration is at least two breaths.
The operations may include resuming, after the set duration, delivery of the breathing gases at the constant operational flow rate for a set interval time. The operations may include after expiration of the set interval time, again reducing the flow rate of the breathing gases to the temporary flow rate. The operations may include while the flow rate is again reduced, capturing second capnography data of exhaled air. The interval time may be at least 50 times greater than the set duration. The operations may further comprise boosting an oxygen concentration level for at least 1 breath immediately prior to reducing the flow rate. The operations may further comprise generating capnography trend data from the first capnography data and the second capnography data. The operations may further comprise, based on the capnography trend data being outside a capnography threshold, activating a notification.

In another aspect, which may be provided independently, the technology relates to a method for capturing capnography data. The method includes delivering breathing gases at a constant operational flow rate and an operational oxygen concentration level, wherein the operational flow rate is greater than 40 liters per minute (lpm); reducing, for a set duration, a flow rate of the breathing gases to a temporary flow rate, wherein the flow rate is at least 10 lpm less than the operational flow rate; while the flow rate is reduced, capturing first capnography data for exhaled air; resuming, after the set duration, delivery of the breathing gases at the constant operational flow rate for a set interval time; after expiration of the set interval time, again reducing the flow rate of the breathing gases to the temporary flow rate; and while the flow rate is again reduced, capturing second capnography data of exhaled air.

In an example, the interval time is at least 50 times greater than the set duration. In another example, the method further includes boosting an oxygen concentration level for at least 1 breath immediately prior to reducing the flow rate. In still another example, the method further includes generating capnography trend data from the first capnography data and the second capnography data. In yet another example, the method further includes based on the capnography trend data being outside a capnography threshold, activating a notification.

In a further aspect, which may be provided independently, there is provided a capnography monitor configured to be communicatively coupled to a valve, the capnography monitor comprising:
a processor; and
   memory storing instructions that, when executed by the processor, cause the capnography monitor to perform a set of operations comprising:
outputting a control signal to at least partially close the valve for a set duration to reduce a flow rate of breathing gases delivered to the patient;
during the set duration, capturing capnography data for exhaled air; and
upon expiration of the set duration, outputting a further control signal to reopen the valve to allow the flow rate of breathing gases delivered to the patient to increase.

In a further aspect, which may be provided independently, there is provided a computer program product comprising computer-readable instructions that are executable by a processor to perform a set of operations comprising:
outputting a control signal to at least partially close a valve for a set duration to reduce a flow rate of breathing gases delivered to a patient;
during the set duration, capturing capnography data for exhaled air; and
upon expiration of the set duration, output a further control signal to reopen the valve to allow the flow rate of breathing gases delivered to the patient to increase.
Features of one aspect or example may be provided as feature of any other aspect or example in any appropriate combination. For example, any one of method, system, monitor or computer program product features may be provided as any one or more other of method, system, monitor or computer program product features.

It is to be understood that both the foregoing general description and the following Detailed Description are explanatory and are intended to provide further aspects and examples of the disclosure as claimed.

### BRIEF DESCRIPTION OF THE DRAWINGS

The following drawing figures, which form a part of this application, are illustrative of aspects of systems and methods described below and are not meant to limit the scope of the disclosure in any manner, which scope shall be based on the claims.
FIG. 1 is a diagram illustrating an example of a medical ventilation system.
FIG. 2 depicts an example capnography monitoring system displaying an unattenuated capnography waveform.
FIG. 3 depicts example capnography waveforms.
FIG. 4 depicts an example capnography monitoring system displaying an attenuated capnography waveform.
FIG. 5 is a flowchart illustrating an example method for measuring capnography data during high-flow oxygen therapy.
FIGS. 6A-6C depict example plots of flow rate and oxygen concentration over time.

While examples of the disclosure are amenable to various modifications and alternative forms, specific aspects have been shown by way of example in the drawings and are described in detail below. The intention is not to limit the scope of the disclosure to the particular aspects described. On the contrary, the disclosure is intended to cover all modifications, equivalents, and alternatives falling within the scope of the disclosure and the appended claims.

### DETAILED DESCRIPTION

Medical ventilators are used to provide breathing gases to patients who are otherwise unable to breathe sufficiently. In modern medical facilities, pressurized air and oxygen sources are often available from wall outlets, tanks, or other sources of pressurized gases. Accordingly, ventilators may provide pressure regulating valves (or regulators) connected to centralized sources of pressurized air and pressurized oxygen. The regulating valves function to regulate flow so that respiratory gases having a desired concentration are supplied to the patient at desired pressures and flow rates. Further, as each patient may require a different ventilation strategy, modern ventilators may be customized for the particular needs of an individual patient.

As discussed briefly above, one mode of ventilation is a known as high-flow oxygen therapy (HFOT), where a patient is provided a relatively high flow of breathing gases that includes an increased concentration of oxygen. For example, some HFOT systems are capable of delivering flow rates of up to 80-100 liters per minute (lpm) with oxygen concentrations (e.g., fraction of inspired oxygen (FiO₂) levels) of up to or above 90% or 100%. While HFOT has proven to be particularly useful in treating certain conditions, high flow rates generated during HFOT can cause challenges for accurately measuring breath characteristics of the patient.

Capnography, which measures the amount of carbon dioxide (CO₂) in exhaled air from a patient, is one such area that becomes difficult in HFOT. In capnography, the carbon dioxide levels are from gases at or near the patient's mouth nose. As the patient breathes, the amount of carbon dioxide present is those gases changes. For instance, the amount of carbon dioxide increases as the patient exhales and decreases as the patient inhales because inhaled air generally contains little to no carbon dioxide and the exhaled air may contain about 4-6% carbon dioxide. The ability to accurately measure the amount of carbon dioxide, however, becomes more difficult as the flow of air being provided to the patient increases. For example, the high flow rate in HFOT effectively washes out the carbon dioxide that is exhaled from the patient, which significantly lowers the amount of carbon dioxide that can be measured. The resultant capnography signal or waveform is thus significantly attenuated.

The attenuated capnography signal or waveform may prevent accurate use of the capnography data and reduce its overall clinical value. When the capnography waveform is robust (e.g., unattenuated), significant clinical determinations can be made from the capnography signal. For example, the frequency of the waveform may be calculated to determine a respiratory rate of the patient. The shape of the waveform may also indicate different conditions of the patient, such as apnea, hypoventilation, hyperventilation, rebreathing of carbon dioxide, partial airway obstructions, asthma, emphysema, and/or chronic obstructive pulmonary disease (COPD), among others.

The peak value of the waveform during an exhalation phase, known as the end tidal CO2 (etCO₂) value, also has clinical uses. For instance, a trend of the etCO₂ value over time may indicate improvement or decline in the pulmonary health of a patient. The etCO₂ value itself may also be indicative of potential issues, as a normal healthy patient may have an etCO₂ value between about 35-45 mmHg. When the capnography waveform, however, is attenuated due to washout from the high flow of gases in HFOT, the above uses of the capnography waveform and the etCO₂ value are diminished and/or the accuracy of such uses is reduced.

Among other things, to alleviate the above challenges resulting from carbon dioxide washout from HFOT, the present technology temporarily alters the HFOT delivery to allow for capnography measurements to occur. For example, the present technology may temporarily reduce the flow rate of gases being provided to the patient to allow for capnography measurements to be taken over one or more breaths. During the temporary reduction of flow rate, an increase in oxygen concentration may also be provided to help maintain blood oxygen levels of the patient. In other examples, the oxygen concentration levels may be adjusted prior to the temporary reduction in flow to raise the blood oxygen levels of the patient before the reduction in flow. The duration of the temporary reduction in flow and/or the amount of reduction in flow may be based on the type of use desired for the capnography data measured during the temporary reduction in flow. The duration and amount may also be iteratively adjusted between the temporary reductions based on the captured capnography data. For instance, capnography data may be captured during a first temporary reduction in flow. Based on the characteristics of that captured capnography data, the duration and/or reduction amount during a second temporary reduction in flow may be adjusted.

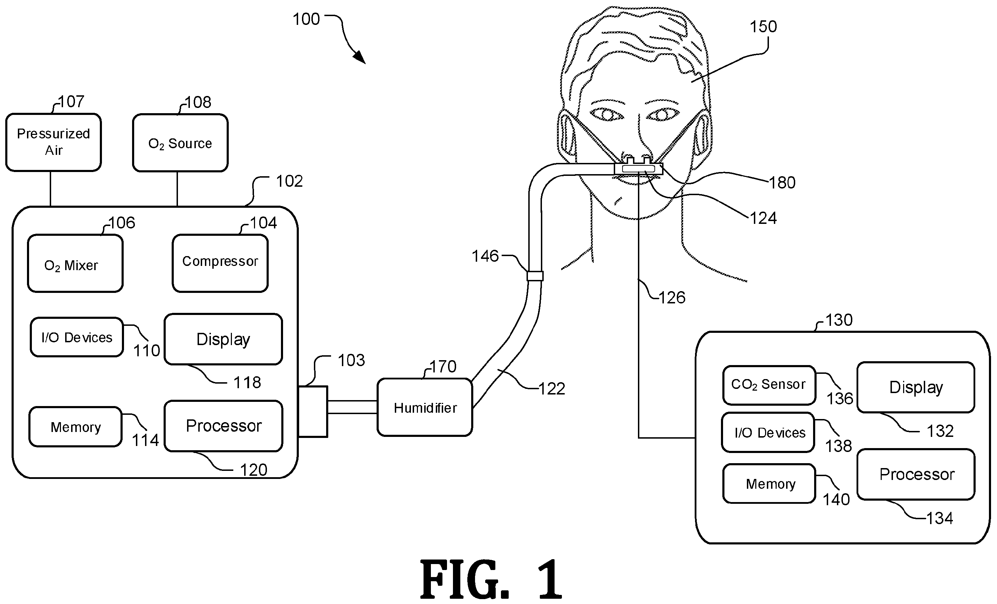

FIG. 1 is a diagram illustrating an example of a medical ventilation system 100. The medical ventilation system 100 includes a medical ventilator 102. The medical ventilator 102 is connected to a human patient 150. The ventilator 102 may provide positive pressure ventilation to the patient 150. For instance, the ventilator 102 may provide HFOT to the patient 150.

The ventilator 102 delivers breathing gases to the patient 150 via ventilation tubing 122. In the example depicted, a single limb circuit is depicted. The single limb of the ventilation tubing 122 that delivers breathing gases to the patient may also be referred to as an inspiratory limb. The inspiratory limb extends from an inspiratory port 103 of the medical ventilator 102. The inspiratory limb extends to a patient interface 180 which couples the patient to the pneumatic system via an invasive (e.g., endotracheal tube) or a non-invasive (e.g., nasal cannula, nasal mask) patient interface 180. In the example depicted, the patient interface 180 is a nasal cannula. As such, when the ventilator 102 delivers HFOT to the patient 150, such ventilation may be referred to as HFNC oxygen therapy. In other examples, a dual limb circuit may be implemented where an expiratory limb extends from the patient interface 180 and returns to an expiratory port of the ventilator 102.

The breathing gases delivered to the patient 150 may be generated from a combination of oxygen and environmental or room air (e.g., ambient air outside of the ventilator 102). The oxygen may be received from an external oxygen source 108. The oxygen source 108 may include pressurized oxygen from a tank or a wall port, such as oxygen ports that are available in many medical facilities. The ambient air may be received from a pressurized air source 107. The pressurized air source 107 may include pressurized air from a tank or a wall port, such as pressurized air ports that are available in many medical facilities. Valves may be included on the ventilator 102 and/or at the pressurized air source 107 and/or the oxygen source 108 to control the flow of air and oxygen into the ventilator 102. Alternatively or additionally, the medical ventilator 102 may include a compressor 104 or blower that pressurizes the air and/or oxygen. In such examples, a pressurized air source 107 may be omitted, and the compressor 104 may draw ambient air in through a vent or ambient air inlet of the ventilator 102. The compressor 104 may be controlled to generate a desired pressure or flow of gases.

An oxygen mixer 106 may also be included in the ventilator 102 to mix the oxygen from the oxygen source 108 and the ambient air to achieve a set oxygen concentration level (e.g., FiO₂ level). The oxygen mixer may include a chamber and/or a series of controllable valves to control the flow of oxygen and/or ambient air to achieve a set oxygen concentration level (e.g., FiO₂ level). The mixture of oxygen and ambient air results in breathing gases that are delivered to the patient 150.

In some examples, the inspiratory port 103 may include a valve, which may be a proportional valve such as a proportional solenoid valve (PSOL). The rate of flow (e.g., the liters per minute) of breathing gases delivered to the patient may then be controlled by operating or adjusting at least one of the pressurized air source 107 (or associated valves), the oxygen source 108 (or associated valves), the compressor 104, valves of the oxygen mixer 106, and/or the valve of the inspiratory port 103.

In some examples, a humidification system 170 may also be incorporated to humidify the breathing gases that are delivered to the patient 105. The humidification system 170 may be coupled to the inspiratory limb of the ventilation tubing such that humidification (e.g., water vapor) is added to the breathing gases as they pass through the humidification system 170.

A coupling 146 may also be incorporated into the ventilation tubing 122 where the inspiratory limb is connected to tubing of the patient interface 180. For instance, the coupling 146 may be a point where the inspiratory limb from the ventilator 102 and/or humidifier 170 connects or meets the tubing from the patient interface 180. The portion of tubing on the patient side of the coupling 146 may be an integrated or detachable portion of the patient interface 180 that connects to the nasal cannula (or other type of patient interface 180).

In some examples, the coupling 146 may also include a valve, which may be a proportional valve, such as a scissor valve or PSOL. In other examples, the valve may be located in another portion of the ventilation tubing 122, such as at or near the inspiratory port 103. The valve may be controlled to initiate the temporary flow-reduction function described herein.

The ventilator 102 may also include a display 118 to display data and/or settings regarding ventilation being delivered to the patient 150. The ventilator 102 may also include one or more processors 120 to execute instructions to the control the ventilator 102 and/or other devices and components of the medical ventilation system 100. The ventilator 102 may also include memory 114 that stores instructions that, when executed by the processor 120, cause the ventilator to perform the operations discussed herein. For instance, the memory 114 includes non-transitory, computer-readable storage media that stores software that is executed by the processor 120 and which controls the operation of the ventilator 102. In an example, the memory 114 includes one or more solid-state storage devices such as flash memory chips. In an alternative example, the memory 114 may be mass storage connected to the processor 120 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 120. That is, computer-readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by the computer. Communication between components of the system 100 or between the system 100 and other therapeutic equipment and/or remote monitoring systems may be conducted over a distributed network, as described further herein, via wired or wireless means.

The ventilator 102 may also include various input and/or output (I/O) devices 110. The I/O devices 110 may include a touchscreen, which may be incorporated into the display 118. The I/O devices may also include speakers for emitting audible signals, such as alarms or other notifications. A keyboard, buttons, a mouse or pointer, or other types of input devices may also be incorporated to allow for input from a user. For instance, the flow and oxygen concentration levels of the delivered breathing gases may be set by a medical professional using the I/O devices 110.

The medical ventilation system 100 may also include a capnography monitor 130. The capnography monitor 130 may receive sampled air via a sampling line 126 that is connected to a sampling interface 124, which may be incorporated into the patient interface 180. For instance, a sampling of air captured at the sampling interface 124 is drawn into the capnography monitor 130 where the air is analyzed for carbon dioxide content. The air may be drawn into the capnography monitor 130 via a vacuum system incorporated into the monitor 130. While not shown in FIG. 1, the sampling interface 124 may also include portions that extend over the patient's mouth, such as an oral scoop, that captures exhaled air from the mouth of the patient 105. In other examples, the carbon dioxide measurement may be made at the sampling interface 124, and an electrical signal representing the measured carbon dioxide amount may be communicated to the capnography monitor 130. Different types of sampling interfaces and capnography devices, such as sidestream, mainstream technology may be implemented.

While the sampling interface 124 is depicted as being at, near, and/or integrated into the patient interface 180 in FIG. 1, the sampling interface 124 may be incorporated in other positions of the breathing circuit or ventilation tubing 122. For instance, the sampling interface 124 may be integrated at or near the coupling 146. In examples where an expiratory limb is also incorporated, the sampling interface 124 may be incorporated into the expiratory limb.

Once the capnography monitor 130 receives the gas sample from the sampling line 126, a carbon dioxide sensor 136 of the capnography monitor 130 measures the carbon dioxide content of the sampled gas. The carbon dioxide sensor 136 may be a variety of sensors suitable for measuring carbon dioxide, such as a sensor that utilizes molecular correlation spectrography. The carbon dioxide measurement may be made as a partial pressure of carbon dioxide in the sample gas. As a result, the carbon dioxide measurement may have units of pressure, such as millimeters of mercury (mmHg).

The measurement operations, calculations, and control operations of the capnography monitor 130 may be performed by or with computing components integrated into the capnography monitor 130. For example, the capnography monitor 130 may include one or more processors 134, I/O devices 138, memory 140, and a display 132. The processor(s) 134 may execute instructions to the control the capnography monitor 130 and/or other devices and components of the medical ventilation system 100. The memory 140 may store instructions that, when executed by the processor 134, cause the ventilator to perform the operations discussed herein. For instance, the memory 114 includes non-transitory, computer-readable storage media that stores software that is executed by the processor 134 and which controls the operation of the capnography monitor 130. The memory 140 may be the same type of memory as the memory 114 of the ventilator 102. The I/O devices 138 and the display 132 may also include the same or similar types of I/O devices 110 and display 118, respectively, of the ventilator 102. For instance, the capnography monitor 130 may include an on/off button, a battery/power indicator, a medium priority alarm, a high priority alarm, an ambient light sensor, a back button, a home button, a menu button, an alarm-silence button, directional buttons, and/or an enter button, among other or alternative features. Accordingly, a medical professional is able to interact with, and change the settings of, the capnography monitor 130.

The capnography monitor 130 may control the initiation of the temporary flow-reduction function discussed herein. For example, the capnography monitor 130 may send a signal to the ventilator 102 to cause the ventilator 102 to reduce the flow and make other related adjustments during the duration of the temporary flow-reduction function. In other examples, the capnography monitor 130 is able to control the valve that is located in the coupling 146 or in another position of the ventilation tubing 122. Accordingly, the capnography monitor 130 may be communicatively coupled to the valve. The capnography monitor 130 may then initiate the temporary flow-reduction function by at least partially closing the valve to reduce the flow of breathing gases reaching the patient. The capnography monitor 130 may then cease the temporary flow-reduction function by reopening the valve.

While the capnography monitor 130 is depicted as being separate from the ventilator 102 in the example system 100, in other examples the capnography monitor 130 may be integrated into the ventilator 102. For example, one or more functionalities and/or components of the capnography monitor 130 may be incorporated into the ventilator 102. Accordingly, the ventilator itself may measure the carbon dioxide amounts and/or output the capnography data.

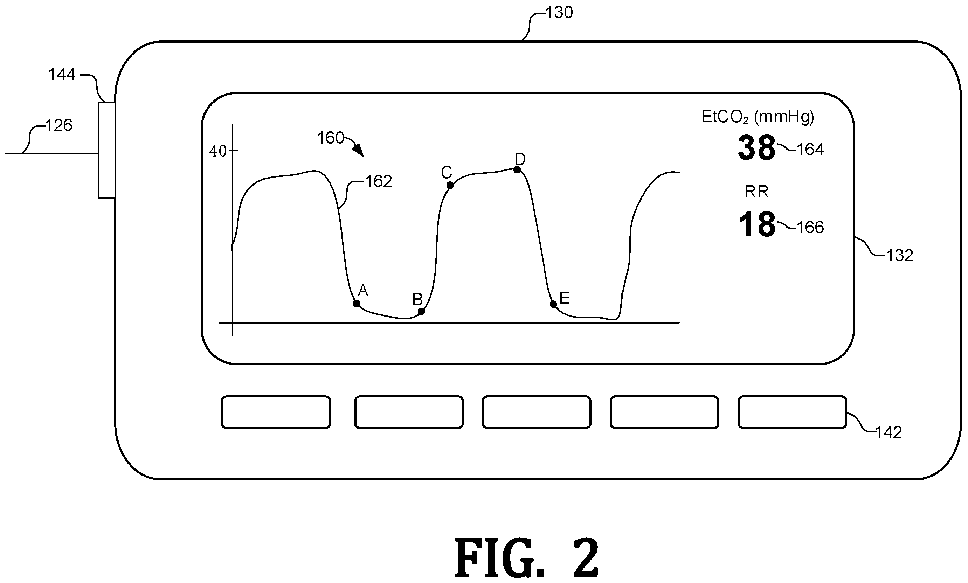

FIG. 2 depicts an example capnography monitoring system 130 displaying an unattenuated capnography waveform 162. The capnography waveform 162 is referred to herein as an "unattenuated" waveform because the waveform is generated from an acceptable breath sample from the patient that is not washed out by high flow rates from a ventilator. For instance, the unattenuated capnography waveform 162 may be captured when the ventilator is delivering a lower flow, or no flow, or gas to the patient.

In the example depicted, one side of the capnography monitor 130 includes an input port 144 for receiving the sampling line 126. The front of the capnography monitor 130 includes the display 132, and a row of buttons 142 are provided below the display 132. The display 132 may display capnography data among other types of data, such as pulse oximetry (e.g., SpO2) data. In FIG. 2, a capnography plot 160 including a capnography waveform 162 is displayed. The y-axis of the capnography plot 160 represents a carbon dioxide level, and in the example depicted, has units of mmHg. The x-axis of the capnography plot 160 represents time.

In addition, an etCO₂ value 164 and a respiratory rate value 166 are displayed. The etCO₂ value 164 and the respiratory rate value 166 may both be calculated or determined by the capnography monitor 130 from the capnography waveform 162. Additional information may also be monitored and/or displayed by the capnography monitor 130, such as pulse oximetry (e.g., SpO₂) data.

Five points (A-E) have been superimposed on the capnography waveform 162 to assist in explaining the various portions of the capnography waveform 162. Phase 1 is defined between points A-B and may be referred to as the inspiratory baseline. During inhalation, little to no carbon dioxide is present at the nose and mouth of the patient. Exhalation begins near point B with air leaving the trachea, posterior pharynx, mouth and nose. The gas which is exhaled from these large conducting airways contains a negligible concentration CO₂. Phase 2 is defined between points B-C and may be referred to as the expiratory upstroke. During this ascending phase, CO₂ rich air from the alveoli begins to reach the upper airway and mix with the dead space air. Phase 3 is defined between points C-D and may be referred to as the alveolar plateau. During Phase 3, the carbon dioxide concentration remains relatively constant as primarily alveolar gas is exhaled. The alveolar plateau may be relatively flat with a slight trend upwards towards the end of the alveolar plateau. The etCO₂ value may be the maximum value of the capnography waveform during Phase 3 or during an exhalation phase of the breath. Phase 4 is defined between points D-E and may be referred to as the start of inhalation. During the start of inhalation, the oxygen fills the airway and CO₂ levels quickly drop back to baseline.

The capnography waveform 162 depicted in FIG. 2 is for a normal patient or a normal capnography waveform. The characteristics of a normal capnography waveform generally includes a somewhat square waveform. For instance, Phase 2 and Phase 4 should be fairly steep and Phase 3 should be fairly flat with some increase during the end of the phase. The respiratory rate value of 18 breaths and the etCO₂ value of 38 are also considered to be within a normal or acceptable range.

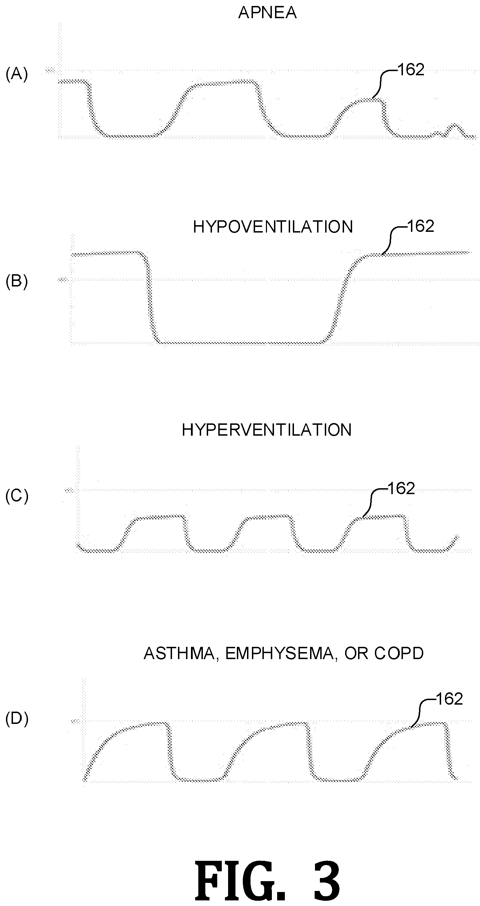

FIG. 3 depicts example capnography waveforms that may be considered abnormal. As discussed above, the shape and characteristics of a capnography waveform may indicate clinical conditions of the patient. The capnography waveforms 162 depicted in FIG. 3 are indicative of different clinical conditions of the patient.

The capnography waveform 162 in plot (A) is indicative of apnea where the etCO₂ value is trending downward for a series of breaths and then no breath occurs for a period of time such as 10 seconds. Causes of apnea may occur due to cardiac arrest, respiratory arrest, equipment failure, a displaced airway adjunct, or obstructive sleep apnea (OSA), among other things.

The capnography waveform 162 in plot (B) is indicative of hypoventilation. The capnography waveform 162 is characterized by a rapid increase at the start of exhalation, smooth and possibly prolonged uploaded during exhalation, and an abrupt descent to a baseline during inhalation. In addition, the etCO2 value from the capnography waveform 162 in plot (B) may be above 45 mmHg, which also may indicate hypoventilation. Causes of hypoventilation may include a decrease in respiratory rate, a decrease in tidal volume, chest compressions, obesity hypoventilation syndrome (OHS), or use of central nervous system (CNS) depressant drugs, among others.

The capnography waveform 162 in plot (C) is indicative of hyperventilation which results in low carbon dioxide level resulting from excessive elimination through rapid or deep breathing or from metabolic acidosis. The shape of the capnography waveform 162 has a rapid increase at the beginning of exhalation, gradual, smooth and possibly shortened or peaked upslope during exhalation, and an abrupt descent to baseline during inhalation. The etCO2 value of the capnography waveform 162 in plot (B) may be below 35 mmHg, which also may indicate hyperventilation. Causes of hyperventilation may include an anxiety/panic disorder, excessive exercise, an increase in respiratory rate, or an increase in tidal volume, among others.

The capnography waveform 162 in plot (D) is indicative of asthma, emphysema, or COPD. The shape or morphology of the capnography waveform 162 is abnormal with a marked Phase 2 to Phase 3 curve with a shark fin appearance and an abrupt descent during Phase 4 back to baseline during inhalation. The shark fin shape is seen in more severe bronchospasm.

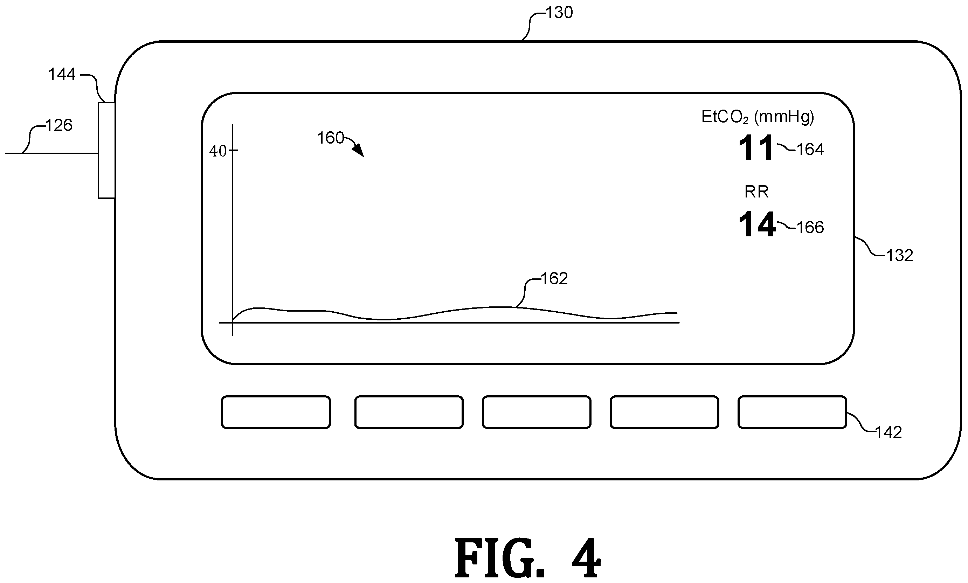

FIG. 4 depicts the example capnography monitoring system 130 displaying an attenuated capnography waveform 163. The attenuated capnography waveform 163 displayed in FIG. 4 results from the exhaled breath of the patient being washed out by the high flow of breathing gases delivered from the ventilator. For example, such an attenuated capnography waveform 163 may be captured when the ventilator is delivering a high flow of gas to the patient, such as 60-80 lpm of breathing gas. As can be seen, the average and maximum amplitude of the attenuated capnography waveform 163 is significantly less than the unattenuated capnography waveform 162. The lower, or attenuated, amplitude is due to the washout of carbon dioxide, exhaled from the patient, due to the high flow of breathing gases being delivered to the patient.

The significant attenuation of the attenuated capnography waveform 163 reduces the clinical usefulness of the capnography waveform 163. For instance, the calculated etCO₂ value 164 is now calculated at 11 mmHg, which would normally be indicative of hyperventilation. But, that low value in this example is due to the carbon dioxide washout rather than a condition of the patient. In addition, the shape of the attenuated capnography waveform 163 is substantially suppressed, which further reduces the possibility of, or the confidence in, matching the shape of the attenuated capnography waveform 163 to one of the waveform shapes known to be indicative of a patient condition, such as the waveforms discussed above in FIG. 3.

Because HFOT involves a constant flow that does not change, or substantially change, during a breath or from breath to breath, the attenuated capnography waveform 163 of FIG. 4 is all that is able to be captured during HFOT therapy. The present technology alleviates the attenuated capnography waveform problem, by temporarily pausing or reducing the flow of breathing gases during HFOT to allow for an unattenuated capnography waveform to be captured during the temporary pause or reduction in flow.

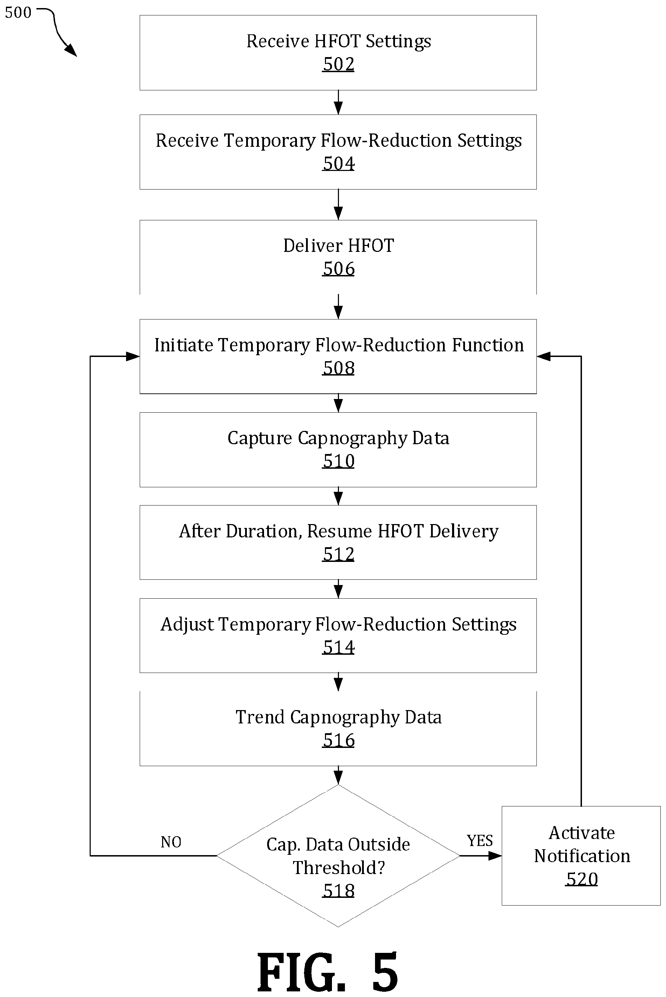

FIG. 5 is a flowchart illustrating an example method 500 for measuring capnography data during high-flow oxygen therapy (HFOT). The example method 500 includes operations that may be implemented or performed by the systems and devices disclosed herein. For example, ventilator 102 (or components thereof) and/or capnography monitor 130 (or components thereof) depicted in FIG. 1 may perform the operations described in the methods. In addition, instructions for performing the operations of the methods disclosed herein may be stored in the memories of the ventilator 102 and/or capnography monitor 130.

At operation 502, settings for the HFOT are received. Among other things, the settings may include a flow rate (e.g., liters per minute) and an oxygen concentration level (e.g., an FiO2 level). The settings may be received from a medical professional via interactions with the ventilator 102. The flow rate to be delivered for the HFOT may be referred to as the operational flow rate, and the oxygen concentration level to be delivered for the HFOT may be referred to as the operational oxygen concentration level. In some examples, default setting values may be stored such that the HFOT may be initiated without requiring additional settings to be received from the medical professional. The medical professional, however, may adjust those default settings values.

At operation 504, settings for a temporary flow-reduction function are received. The settings may be received by a medical professional via interactions with the ventilator 102 and/or the capnography monitor 130. The settings may include how frequently the temporary flow-reduction function should be activated (e.g., a time interval between activations), a duration of the temporary pause function (e.g., a time duration or a number of breaths duration), the flow rate of breathing gases during the temporary pause function (e.g., a flow setting or a percentage reduction in flow), the oxygen concentration (e.g., FiO2 level) during the temporary flow-reduction function, and/or a pre-function oxygen boost setting. The flow rate for the temporary flow-reduction function may be referred to as the temporary flow rate, and the oxygen concentration level for the temporary flow-reduction function may be referred to as the temporary oxygen concentration level.

The pre-function oxygen boost setting may be a binary setting for turning a pre-function oxygen setting on or off. The pre-function oxygen boost setting controls whether the oxygen concentration level is boosted prior to the temporary flow-reduction function being initiated. The pre-function oxygen boost setting may also include underlying settings, such as a percent or value increase in oxygen concentration during the boost and a duration of the oxygen boost. The duration of the boost setting may be based on the set duration of the temporary flow-reduction function. For instance, the duration of the boost may be at least 50% of the duration of the temporary flow-reduction function. In some examples, the boost duration may be between 1-3 breaths.

In some examples, certain settings may be automatically adjusted or set by the ventilator 102 and/or capnography monitor 130 based on other settings received by the medical professional. As an example, a temporary oxygen concentration level may be set based on a duration of the temporary flow-reduction function and/or a temporary flow setting. For instance, as the duration increases and/or the flow decreases, less oxygen is being delivered to the patient during the temporary flow-reduction function. Accordingly, a temporary oxygen concentration level may be automatically increased for longer durations or lower temporary flow settings. For similar reasons, maximums may be provided for some of the settings that do not allow a medical physician to adjust the setting beyond the maximum. For instance, the duration of the temporary flow-reduction function may have a maximum value to prevent under-delivery of breathing gases.

In some examples, default setting values may be stored such that the temporary flow reduction may be initiated without requiring additional settings to be received from the medical professional. The medical professional, however, may adjust those default settings values.

At operation 506, the HFOT is delivered to the patient according to the settings received in operation 502. For instance, a constant flow of breathing gases is delivered at the operational flow rate and the operational oxygen concentration level. As example, breathing gases may be delivered at constant operational flow rate of greater than 40 lpm may be delivered over a plurality of breaths, such as at least 10 or 100 breaths.

A temporary flow-reduction function is initiated at operation 508 according to the settings received in operation 504. The temporary flow-reduction function may be initiated after an interval setting, such as interval settings received in operation 504. In other examples, the temporary flow-reduction function may be initiated in response to receiving a selection (such as a button or user interface element) to activate the temporary flow-reduction function. Initiating the flow-reduction function includes reducing the flow rate of the delivered breathing gases and implementing other settings where utilized, such as changing oxygen concentration levels. For instance, initiating the temporary flow-reduction function may include reducing the flow rate of breathing gases to the temporary flow rate for a set duration and, in some examples, increasing the oxygen concentration level to the temporary oxygen concentration level. Reducing the flow rate of the delivered breathing gases may include reducing the flow rate by at least 10, 20, or 30 lpm.

Initiating the temporary flow-reduction function may be performed by the ventilator 102 and/or the capnography monitor 130. For example, where the settings for the temporary flow-reduction function are received at the ventilator 102 and the capnography functions are also incorporated within the ventilator 102, the ventilator 102 may initiate the temporary flow-reduction function directly. In other examples, where the settings for the temporary flow-reduction function are received at the capnography monitor 130, the capnography monitor 130 send a signal to the ventilator 102 to initiate and/or cease the temporary flow-reduction function.

In yet other examples, where the settings for the temporary flow-reduction function are received at the capnography monitor 130, the capnography monitor 130 may control initiation and cessation of the temporary flow-reduction function directly by controlling a valve coupled to the ventilation tubing 122 (such as at the coupling 146). For example, the capnography monitor 130, after the set interval or upon receipt of an activation selection, at least partially closes the valve to initiate the temporary flow-reduction function. After a set duration from initiation, the capnography monitor 130 reopens the valve to cease the temporary flow-reduction function.

At operation 510, capnography data is captured during the temporary flow-reduction function (e.g., while the flow rate is reduced). The capnography data may be captured by sampling the air around the nose and/or mouth of the patient, as discussed above. The sampling of the capnography data may be performed by the capnography monitor 130 and/or the ventilator 102 when such capabilities are incorporated into the ventilator 102. For instance, capturing the capnography data may include drawing air from a sampling interface 124 through a sampling line 126 and analyzing the sampled air with a carbon dioxide sensor 136. The captured capnography data may include a measurement of the partial pressure of carbon dioxide present in the sampled air over a period of time. Capturing the capnography data in operation 510 may also include generating capnography waveforms, calculating etCO2 values, calculating respiratory rates, and/or determining other values or patient conditions from the capnography data. The capnography data and/or generated data may also be displayed in operation 510. In some examples, the capnography data is captured only during the temporary flow-reduction function. For instance, the capnography data is not captured during delivery of breathing gases at the constant operational flow rate. In other examples, the capnography data is continuously captured during the HFOT delivery and the temporary flow-reduction function.

At operation 512, the after a set duration, the temporary flow-reduction function is ceased and the HFOT delivery is resumed according to the settings received in operation 502. The set duration may be an amount of time or a number of breaths. In examples, where the duration is a number of breaths, the passage of the breaths may be determined from the capnography data, such as the capnography waveform, itself. For instance, as discussed above, the exhalation and inhalation phases of a breath may be identified from the capnography waveform. Thus, the occurrence of a number of breaths may be determined from, or based on, the capnography data. Resuming the HFOT delivery (and/or ceasing the temporary flow-reduction function) may be controlled by the ventilator 102 and/or the capnography monitor 130 in the same or similar manner as described above for initiating the temporary flow-reduction function in operation 508.

At operation 514, the settings for the temporary flow-reduction function may be adjusted. The adjustments to the settings may be automatically made based on a machine analysis of the capnography data captured in operation 510. The ventilator 102 and/or capnography monitor 130 may analyze the capnography waveform generated from the capnography data captured in operation 510 to determine the fidelity of the capnography data. As an example, the shape of each phase of the waveform may be analyzed to determine whether the shape of the respective phase matches what is expected or considered to be a high-fidelity waveform. For instance, in some examples, Phase 2 and Phase 4 should be fairly steep and Phase 3 should be fairly flat with some increase during the phase. If the captured capnography data does not produce such a capnography waveform, then the temporary flow rate may be automatically adjusted to be lower in a subsequent temporary flow-reduction function to further reduce the attenuation of the capnography waveform due to washout from the high flow breathing gases.

Alternatively or additionally, the adjustments to the settings may be made in response receiving adjustment input at the ventilator 102 and/or the capnography monitor 130. For instance, upon reviewing the capnography data captured in operation 510, a medical professional may determine that the temporary flow rate, duration, or any other setting may need to be adjusted. The medical professional may then provide adjustment input into the ventilator 102 and/or the capnography monitor 130 to adjust the settings of the temporary flow-reduction function. The ventilator 102 and/or the capnography monitor 130 then adjusts the settings based on the received input.

In examples where the capnography monitor 130 controls a valve to initiate and cease the temporary flow-reduction function, the adjustment to settings may be an adjustment to a valve position settings when the temporary flow-reduction function is initiated. For example, by closing the valve further (e.g., a more closed position), the delivered flow rate is further reduced. Thus, the respective setting may not need to be a temporary flow rate setting as the actual flow rate value is not needed. Rather the respective setting may be a valve position setting, such as percentage of closure.

At operation 516, a trend of capnography data is generated. As an example, a trend of the capnography data may include generating a trend for the etCO2 values calculated from the capnography data captured during the temporary flow-reduction functions. For instance, an etCO2 value may be calculated for each breath during a temporary flow-reduction function. Multiple temporary flow-reduction functions may be performed during ventilation of a patient. The calculated etCO2 values from the multiple temporary flow-reduction function may be compared to determine or generate a trend of the etCO2 values. For example, an etCO2 value from a first temporary flow-reduction function (e.g., an average etCO2 value) may be compared to an etCO2 value from a second temporary flow-reduction function. In some examples, the trend may be displayed as plot showing the trend of the etCO2 values. Alternatively or additionally, a number or indicator (color, arrow, etc.) may be displayed to indicate the trend of the etCO2 values (e.g., upward, downward, and/or magnitude of trend).

At operation 518, the capnography data (or data generated therefrom) is compared to one or more capnography thresholds to determine whether the capnography data is within acceptable limits. For instance, the raw capnography data, the capnography waveforms, the etCO2 values, the respiratory rates, and/or the trends generating operation 516 may be compared to one or more capnography thresholds. If the capnography data is not outside of the capnography threshold(s) (e.g., within the limits), method 500 flows back to operation 508 where another temporary flow-reduction function is initiated after an interval period (or in response to a selection to initiate the temporary flow-reduction function). Method 500 then repeats from operation 508.

If, at operation 518, the capnography data is outside the capnography threshold(s), a notification is activated or delivered. The notification may be an alarm to indicate a clinical condition of the patient and/or to alert a medical professional that assistance or review may be needed. The notification may be displayed on a display of the ventilator 102 and/or the capnography monitor 130. The notification may also or alternatively be sounded on a speaker of the ventilator 102 and/or the capnography monitor 130. One or more indicators or lights on the ventilator 102 and/or the capnography monitor 130 may also or alternatively be illuminated. The notification may also be communicated or delivered to another device, such as a central monitoring station and/or a device of a medical professional to alert the medical professional. After operation 520 is performed, method 500 flows back to operation 508 where another temporary flow-reduction function is initiated after an interval period (or in response to a selection to initiate the temporary flow-reduction function). Method 500 then repeats from operation 508.

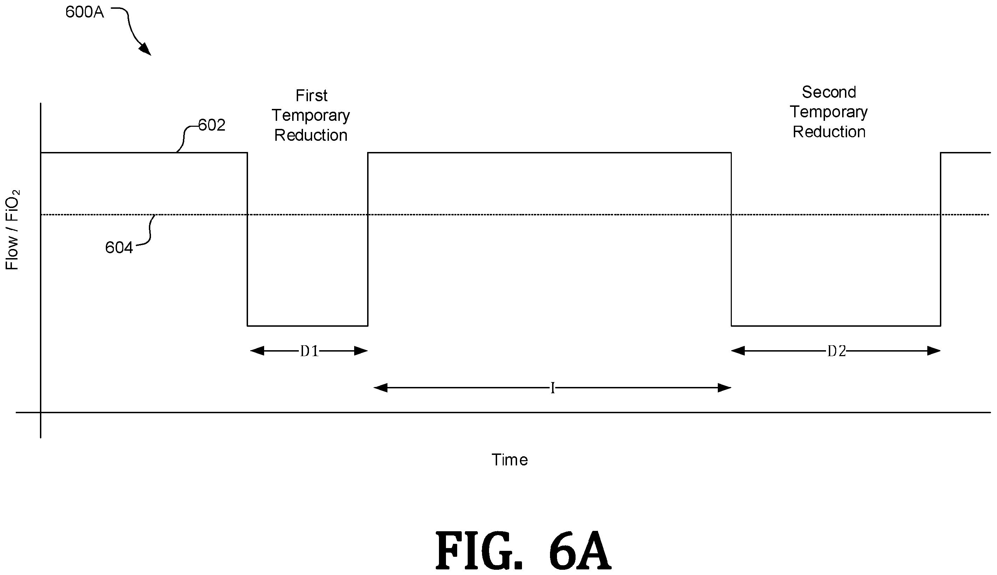

FIGS. 6A-6C depict example plots 600A-C of flow rate 602 and oxygen concentration 604 over time. In the plots 600A-C, time is represented on the x-axis and the magnitude of the flow rate and the oxygen concentration are represented on the y-axis. Each of the plots include periods of HFOT delivery and two temporary flow-reduction functions.

FIG. 6A depicts an example where the settings of the temporary flow-reduction function are adjusted after a first temporary flow-reduction to increase the duration of the temporary flow-reduction function. As can be seen from the plot 600A, at the beginning of the HFOT, the delivered flow rate 602 is at an operating flow rate, and the oxygen concentration level is at an operating oxygen concentration level. The operating flow rate may be between 40-80 lpm in some examples. For purposes of this example, the operating flow rate is 60 lpm and the operating oxygen concentration level is 65%.

When a first temporary flow-reduction function is initiated, the delivered flow rate 602 drops to the temporary flow rate setting, which is in this example is 20 lpm. In other examples, the temporary flow rate may be at least 10 lpm, 20 lpm, or 30 lpm less than the operating flow rate. The temporary flow rate may also be less than 80%, 60%, 50%, 40%, 30%, or 20% of the operating flow rate. As discussed above, a lower temporary flow rate reduces the attenuation of the resultant capnography waveform. In the example depicted in FIG. 6A, the oxygen concentration level remains unchanged during the first temporary flow-reduction function.

The first temporary flow-reduction function has a first duration (D1). The duration of the temporary flow-reduction function may be between 1-10 or 2-7 breaths in some examples. The duration may also be based on the temporary flow rate. For example, for a temporary flow rate between 20-40 lpm (or lower), a duration of 2-3 breaths may be appropriate. For a higher temporary flow rate between 50-60 lpm, a longer duration of 5-6 breaths may be appropriate because the capnography data is more attenuated with the higher flow rate.

At the end of the duration (D1), the temporary flow-reduction function ceases and the HFOT delivery resumes. When the HFOT delivery resumes, the flow rate increases from the temporary flow rate to the operational flow rate. The HFOT delivery resumes for a set interval period (I).

Based on the capnography data captured during the first temporary flow-reduction period, the duration setting for the temporary flow-reduction function is adjusted. More specifically, the duration is increased from D1 to D2. In the depicted example, the duration is increased by two breaths. For instance, additional breaths may have been desired to better understand the characteristics of the capnography data and/or the capnography waveform. After a set interval time (I), a second temporary flow-reduction function is initiated for the adjusted or second duration D2.

As should be appreciated, the plots 600A-C may not be drawn to scale. For instance, while the duration (D1) is depicted as being roughly 1/3 of interval time, in many examples the interval time (e.g., the time where HFOT is being delivered) may be significantly greater than the duration of the temporary flow-reduction function. As an example, in some implementations the interval period may be 10 minutes or greater, whereas the duration of the temporary flow-reduction function may be a few breaths (e.g., 5-20 seconds). In some examples, the internal between temporary flow-reduction functions may be at least 20, 50, 100, 150, or 200 times greater than the duration of the temporary flow-reduction function.

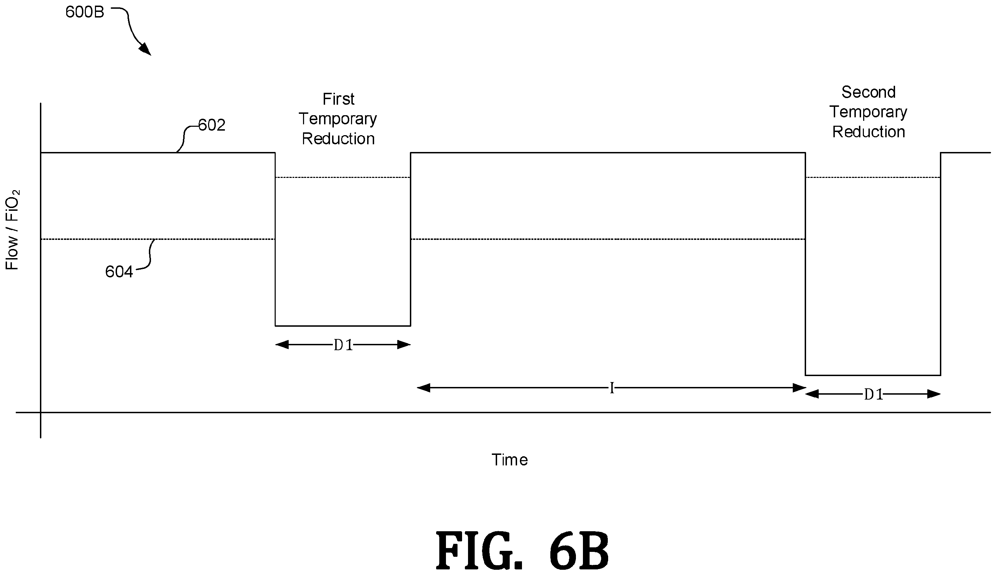

FIG. 6B depicts an example where the settings of the temporary flow-reduction function are adjusted after a first temporary flow-reduction period to lower the temporary flow rate of the temporary flow-reduction function. In addition, in FIG. 6B, the temporary oxygen concentration is higher than the operational oxygen concentration. Accordingly, when the temporary flow-reduction function is initiated, the oxygen concentration level 604 increases and the delivered flow rate 602 decreases. In some examples, the temporary oxygen concentration may be 10-30% higher than the operational oxygen concentration. For instance, where the operational oxygen concentration is and FiO2 level of 60%, the temporary oxygen concentration level may be between 70-90%.

In FIG. 6B, the temporary flow rate setting is lowered after the first temporary flow-reduction function. Thus, upon initiating the second temporary flow-reduction function, the delivered flow rate 602 is dropped below delivered flow rate during the first temporary flow-reduction function.

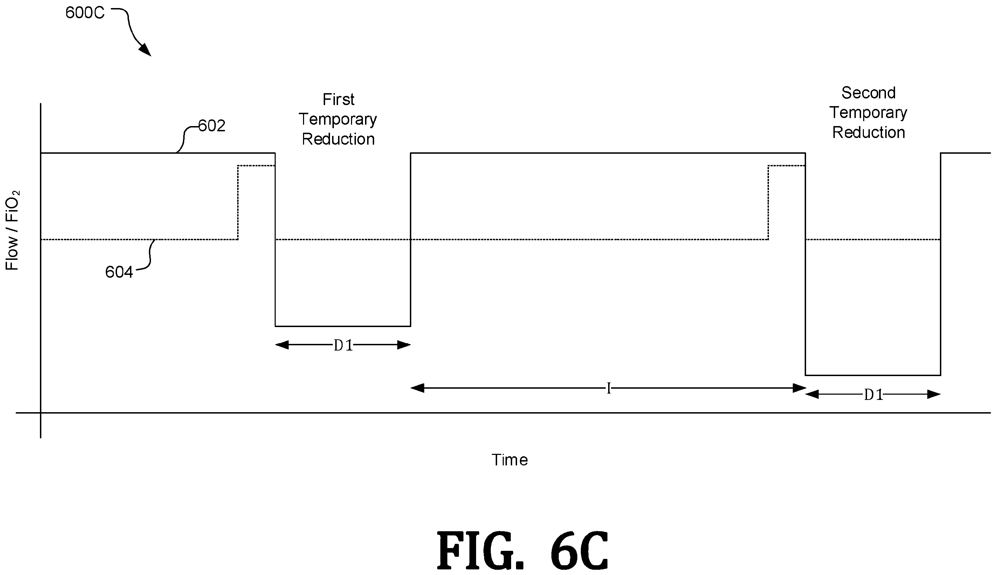

FIG. 6C depicts an example where a pre-function oxygen boost is implemented. Prior to the temporary flow-reduction function being implemented, the oxygen concentration level is increased or a boosted. The boost of oxygen may be for a few breaths (e.g., 1-5 breaths) immediately prior to the initiation of the temporary flow-reduction function. The pre-function oxygen boost may also occur prior to every temporary flow-reduction function, such as the first and the second temporary flow-reduction function depicted in FIG. 6B. In some examples, the boosted oxygen level may be at least 10-30% higher than the operational oxygen concentration. For instance, where the operational oxygen concentration is and FiO2 level of 60%, the boosted oxygen concentration level may be between 70-90%. When the temporary flow-reduction function is initiated, the delivered oxygen concentration level drops to the temporary oxygen level, which in the example depicted, is the same as the operational oxygen concentration level.

A person of skill in the art will understand that the technology described in the context of a medical ventilator for human patients could be adapted for use with other systems such as ventilators for non-human patients or general gas transport systems. Additionally, a person of ordinary skill in the art will understand that the temporary flow-reduction function may be implemented in a variety of breathing circuit setups.

Those skilled in the art will recognize that the methods and systems of the present disclosure may be implemented in many manners and as such are not to be limited by the foregoing aspects and examples. In other words, functional elements being performed by a single component or multiple components, in various combinations of hardware and software or firmware, and individual functions, can be distributed among software applications at either the client or server level or both. In this regard, any number of the features of the different aspects described herein may be combined into single or multiple aspects, and alternate aspects having fewer than or more than all of the features herein described are possible.

Functionality may also be, in whole or in part, distributed among multiple components, in manners now known or to become known. Thus, a myriad of software/hardware/firmware combinations are possible in achieving the functions, features, interfaces and preferences described herein. Moreover, the scope of the present disclosure covers conventionally known manners for carrying out the described features and functions and interfaces, and those variations and modifications that may be made to the hardware or software firmware components described herein as would be understood by those skilled in the art now and hereafter. In addition, some aspects of the present disclosure are described above with reference to block diagrams and/or operational illustrations of systems and methods according to aspects of this disclosure. The functions, operations, and/or acts noted in the blocks may occur out of the order that is shown in any respective flowchart. For example, two blocks shown in succession may in fact be executed or performed substantially concurrently or in reverse order, depending on the functionality and implementation involved.

Further, as used herein and in the claims, the phrase "at least one of element A, element B, or element C" is intended to convey any of: element A, element B, element C, elements A and B, elements A and C, elements B and C, and elements A, B, and C. In addition, one having skill in the art will understand the degree to which terms such as "about" or "substantially" convey in light of the measurement techniques utilized herein or used by those having skill in the medical ventilation art. To the extent such terms may not be clearly defined or understood by one having skill in the art, the term "about" shall mean plus or minus ten percent.

Numerous other changes may be made which will readily suggest themselves to those skilled in the art and which are encompassed in the appended claims. While various aspects have been described for purposes of this disclosure, various changes and modifications may be made which are well within the scope of the disclosure.

## Claims

1. A system (100) for capturing capnography data during high-flow oxygen therapy (HFOT), the system comprising:
a valve coupled to ventilation tubing (122), the valve configured to alter a flow of breathing gases delivered to a patient (150) from a ventilator (102) at an operational flow rate and an operational oxygen concentration level; and
a capnography monitor (130) communicatively coupled to the valve, the capnography monitor comprising:
a processor (134); and
memory storing instructions (140) that, when executed by the processor, cause the capnography monitor to perform a set of operations comprising:
at least partially closing the valve for a set duration to reduce a flow rate of breathing gases delivered to the patient;
during the set duration, capturing capnography data for exhaled air; and
upon expiration of the set duration, reopening the valve to allow the flow rate of breathing gases delivered to the patient to increase,
wherein the system is configured to boost an oxygen concentration level in the breathing gases for at least one breath immediately prior to the set duration.

2. The system of claim 1, wherein capturing the capnography data comprises sampling the exhaled air at a sampling interface (124) and drawing the exhaled air into the capnography monitor through a sampling line (126).

3. The system of claim 1 or 2, wherein the operations further comprise comparing the capnography data to a capnography threshold, and activating a notification when the capnography data exceeds the capnography threshold.

4. The system of any of claims 1 to 3, wherein at least partially closing the valve reduces the flow rate of breathing gases by at least 20 liters per minute, optionally at least 40 liters per minute.

5. The system of any of claims 1 to 4, wherein the set duration is at least two breaths, optionally at least three breaths.

6. The system of any of claims 1 to 5, wherein the boosted oxygen concentration level in the breathing gases is at least 10% higher than the operational oxygen concentration level.

7. The system of any of claims 1 to 6, wherein, during the set duration, the breathing gases are delivered at a temporary oxygen concentration level that is greater than the operational oxygen concentration level.

8. The system of any of claims 1 to 7, wherein the system is configured to, based on the captured capnography data, automatically adjust the temporary flow rate or the set duration for a subsequent temporary flow-reduction function.

9. The system of any of claims 1 to 8, wherein the system is configured to generate capnography trend data from the capnography data and, based on the capnography trend data being outside a capnography threshold, activate a notification.

10. The system of any of claims 1 to 9, wherein the system is configured to calculate an end-tidal carbon dioxide value from the captured capnography data.

## Patentansprüche

1. System (100) zur Erfassung von Kapnographiedaten während einer Sauerstofftherapie mit hohem Durchfluss (HFOT), wobei das System umfasst:
ein Ventil, das an Beatmungsschläuche (122) gekoppelt ist, wobei das Ventil konfiguriert ist, um einen Strom von Atemgasen zu verändern, die einem Patienten (150) von einem Beatmungsgerät (102) bei einer Betriebsströmungsgeschwindigkeit und einem Betriebssauerstoffspiegel zugeführt werden; und
einen Kapnographiemonitor (130), der kommunikativ an das Ventil gekoppelt ist, wobei der Kapnographiemonitor umfasst:
einen Prozessor (134); und
Speicher, der Anweisungen (140) speichert, die bei Ausführung durch den Prozessor bewirken, dass der Kapnographiemonitor eine Reihe von Vorgängen durchführt, umfassend:
mindestens teilweises Schließen des Ventils für eine festgelegte Dauer, um eine Strömungsgeschwindigkeit von an den Patienten abgegebenen Atemgasen zu reduzieren;
während der eingestellten Dauer, Erfassen von Kapnographiedaten für ausgeatmete Luft; und
nach Ablauf der eingestellten Dauer, Wiederöffnen des Ventils, um zu ermöglichen, dass die Strömungsgeschwindigkeit der an den Patienten abgegebenen Atemgase ansteigt,
wobei das System konfiguriert ist, um einen Sauerstoffspiegel in den Atemgasen für mindestens einen Atemzug unmittelbar vor der festgelegten Dauer zu erhöhen.

2. System nach Anspruch 1, wobei das Erfassen der Kapnographiedaten das Probenehmen der ausgeatmeten Luft an einer Probenahmeschnittstelle (124) und das Ziehen der ausgeatmeten Luft in den Kapnographiemonitor durch eine Probenahmestrecke (126) umfasst.

3. System nach Anspruch 1 oder 2, wobei die Vorgänge ferner das Vergleichen der Kapnographiedaten mit einem Kapnographieschwellenwert und das Aktivieren einer Benachrichtigung umfassen, wenn die Kapnographiedaten den Kapnographieschwellenwert überschreiten.

4. System nach einem der Ansprüche 1 bis 3, wobei das mindestens teilweise Schließen des Ventils die Strömungsgeschwindigkeit von Atemgasen um mindestens 20 Liter pro Minute, optional um mindestens 40 Liter pro Minute, reduziert.

5. System nach einem der Ansprüche 1 bis 4, wobei die eingestellte Dauer mindestens zwei Atemzüge, optional mindestens drei Atemzüge beträgt.

6. System nach einem der Ansprüche 1 bis 5, wobei der erhöhte Sauerstoffspiegel in den Atemgasen mindestens 10 % höher ist als der Betriebssauerstoffspiegel.

7. System nach einem der Ansprüche 1 bis 6, wobei während der festgelegten Dauer die Atemgase bei einem temporären Sauerstoffspiegel abgegeben werden, der größer ist als der Betriebssauerstoffspiegel.

8. System nach einem der Ansprüche 1 bis 7, wobei das System konfiguriert ist, um basierend auf den erfassten Kapnographiedaten automatisch die temporäre Strömungsgeschwindigkeit oder die eingestellte Dauer für eine nachfolgende temporäre Strömungsreduktionsfunktion anzupassen.

9. System nach einem der Ansprüche 1 bis 8, wobei das System konfiguriert ist, um Kapnographietrenddaten aus den Kapnographiedaten zu erzeugen und, basierend darauf, dass die Kapnographietrenddaten außerhalb eines Kapnographieschwellenwerts liegen, eine Benachrichtigung zu aktivieren.

10. System nach einem der Ansprüche 1 bis 9, wobei das System konfiguriert ist, um einen endexspiratorischen Kohlendioxidwert aus den erfassten Kapnographiedaten zu berechnen.

## Revendications

1. Système (100) permettant de capturer des données de capnographie pendant une oxygénothérapie à haut débit (HFOT), le système comprenant :
une soupape accouplée à un tuyau de ventilation (122), la soupape étant conçue pour modifier un flux de gaz respiratoires administré à un patient (150) à partir d'un ventilateur (102) à un débit opérationnel et à un niveau de concentration en oxygène opérationnel ; et
un moniteur de capnographie (130) couplé en communication à la soupape, le moniteur de capnographie comprenant :
un processeur (134) ; et
une mémoire stockant des instructions (140) qui, lorsqu'elles sont exécutées par le processeur, amènent le moniteur de capnographie à effectuer une série d'opérations comprenant :
la fermeture au moins partiellement de la soupape pendant une durée définie pour réduire un débit de gaz respiratoires administrés au patient ;
pendant la durée définie, la capture de données de capnographie pour l'air exhalé ; et
à l'expiration de la durée définie, la réouverture de la soupape pour permettre au débit des gaz respiratoires administrés au patient d'augmenter,
dans lequel le système est configuré pour augmenter un niveau de concentration en oxygène dans les gaz respiratoires pour au moins une respiration immédiatement avant la durée définie.

2. Système selon la revendication 1, dans lequel la capture des données de capnographie comprend l'échantillonnage de l'air exhalé au niveau d'une interface d'échantillonnage (124) et l'aspiration de l'air exhalé dans le moniteur de capnographie par le biais d'une ligne d'échantillonnage (126).

3. Système selon la revendication 1 ou 2, dans lequel les opérations comprennent en outre la comparaison des données de capnographie à un seuil de capnographie, et l'activation d'une notification lorsque les données de capnographie dépassent le seuil de capnographie.

4. Système selon l'une quelconque des revendications 1 à 3, dans lequel la fermeture au moins partiellement de la soupape réduit le débit des gaz respiratoires d'au moins 20 litres par minute, éventuellement d'au moins 40 litres par minute.

5. Système selon l'une quelconque des revendications 1 à 4, dans lequel la durée définie est d'au moins deux respirations, éventuellement d'au moins trois respirations.

6. Système selon l'une quelconque des revendications 1 à 5, dans lequel le niveau de concentration en oxygène augmenté dans les gaz respiratoires est au moins 10 % supérieur au niveau de concentration en oxygène opérationnel.

7. Système selon l'une quelconque des revendications 1 à 6, dans lequel, pendant la durée définie, les gaz respiratoires sont administrés à un niveau de concentration en oxygène temporaire qui est supérieur au niveau de concentration en oxygène opérationnel.

8. Système selon l'une quelconque des revendications 1 à 7, dans lequel le système est configuré pour, en fonction des données de capnographie capturées, ajuster automatiquement le débit temporaire ou la durée définie pour une fonction de réduction de débit temporaire ultérieure.

9. Système selon l'une quelconque des revendications 1 à 8, dans lequel le système est configuré pour générer des données de tendance de capnographie à partir des données de capnographie et, en fonction du fait que les données de tendance de capnographie sont en dehors d'un seuil de capnographie, activer une notification.

10. Système selon l'une quelconque des revendications 1 à 9, dans lequel le système est configuré pour calculer une valeur de dioxyde de carbone télé-expiratoire à partir des données de capnographie capturées.
